# EUROPEAN PATENT SPECIFICATION

(11) **EP 3 813 907 B1**
(45) Date of publication and mention of the grant of the patent: **11.09.2024**
(21) Application number: 19731767.0
(22) Date of filing: 24.06.2019
(51) Int. Cl.: A61M 5/30

(54) **NEEDLELESS INJECTION SYSTEM**
NADELLOSES EINSPRITZSYSTEM
SYSTÈME D'INJECTION SANS AIGUILLE

(30) Priority: 26.06.2018 FR 1855736
(43) Date of publication of application: 05.05.2021
(73) Proprietor: L'OREAL, 75008 Paris (FR)
(72) Inventor: PLANARD-LUONG, Lien, 94152 Chevilly La Rue (FR); GILLANT, Flavie, 94152 Chevilly La Rue (FR)
(74) Representative: Cabinet Nony
(86) International application number: PCT/EP2019/066712
(87) International publication number: WO 2020/002262

(56) References cited:
- EP-A1- 2 722 008
- WO-A1-2011/029572
- US-A1- 2009 240 230

## Description

The present invention relates to a needleless injection system and to a method for the treatment, notably cosmetic treatment, of human keratin materials.

### Prior art

Publications US 8876758 B2, US 9186461 B2, US 8066661 B2, US 20160129191 A1, US 8734384 B2 and US 9265888 B2 disclose needleless injectors comprising a reservoir containing a gas, a chamber containing a piston and a viscous liquid formulation to be injected, the chamber communicating with an injection nozzle.

Once the chamber is empty, such needleless injectors require it to be replaced or refilled, notably manually, so that they can become usable again.

Such needleless injectors have an injection nozzle, the geometry and dimensions of which are fixed, thereby limiting the formulations that can be used, notably in terms of viscosity. This leads to restrictions on the treatments that can be performed.

For example, there are various products currently available on the market for treating wrinkles, such as hyaluronic acid or Botox, which have different rheologies. Thus, one and the same needleless injector as described above does not permit injection of both these products since the geometry and the dimensions of the injection nozzle, notably the size of its outlet orifice, need to be tailored to suit the product to be injected.

Furthermore, publication US 2009/0240230 A1 discloses a needleless injector comprising a hand piece comprising a cartridge of a fluid to be injected and an injection nozzle communicating with the cartridge. Such an injector further comprises a pressurized gas reservoir that actuates a propulsion mechanism for injecting the fluid. This publication mentions the possibility, prior to injection, of cooling or heating the surface of the skin at the injection site, but does not specify how this is done.

There is still a need to further improve the injection of a product into human keratin materials via a needleless injector, notably in order to prevent, treat and/or reduce a relief and/or a fold in an area of skin.

### Summary of the invention

The invention responds to this need by virtue of a needleless injection system for injecting a composition, the system comprising and/or designed to receive a reservoir containing a pressurized gas, notably stored in gaseous and/or liquefied form, the system comprising conveying ducts, enabling the gas to be directed,
first, towards the human keratin materials in order to cool said materials by means of the cold generated by an expansion of the pressurized gas and/or towards a surface configured such as to capture the cold generated by an expansion of the pressurized gas and to transmit it towards the human keratin materials in order to cool the human keratin materials,
and, second, towards a mechanism for propelling the composition into the human keratin materials using the gas pressure.

"Needleless injection system" should be understood as being a system in which mechanical forces are used to propel a composition through the epidermis without the penetration of an injection needle through the epidermis.

"Pressurized gas" is understood to mean a compressed gas stored in gaseous and/or liquefied form at a pressure suited to the application sought.

The system according to the invention offers the advantage of it being possible to generate cold easily by utilizing the presence of the pressurized gas used to operate the propulsion mechanism. This cold may allow cooling of human keratin materials at the injection site or around the injection site, which makes it possible, notably, to reduce the potential pain sensation that may be felt by the user during treatment. Cooling is all the more effective when a plurality of successive expansions are carried out closely together over time, which may occur when the expansion used for cooling and the expansion used for injection take place concomitantly.

"Skin" denotes the dermis and the epidermis and the surface region of the mucous membranes, such as the lips.

The propulsion mechanism may comprise an actuating chamber, a thrust element, a push rod and a first piston.

The propulsion mechanism may be actuated by the gas pressure that moves the thrust element, the push rod and/or the first piston.

Preferably, the system according to the invention comprises a hand piece comprising and/or designed to receive the reservoir containing the pressurized gas. This makes it possible to use a compact injection system.

The system according to the invention may comprise the composition to be injected.

The system may comprise a thermal pad cooled by the cold generated by the expansion of the pressurized gas and configured such as to come lean on the human keratin materials at the injection site or around the injection site.

This thermal pad may be of annular form, injection then being carried out through the pad.

The system may comprise at least one thermal bridge within or in contact with which the pressurized gas expands, the or each thermal bridge being configured such as to convey the cold generated by the expansion of the pressurized gas to the thermal pad.

Preferably, expansion of the pressurized gas takes place in the hand piece. This makes it possible to generate cold as closely as possible to the keratin materials.

Preferably, the or each thermal bridge and the thermal pad are made from a heat-conducting material, notably a metallic material comprising aluminium, copper and/or silver. Preferably, the heat-conducting material is in aluminium or an aluminium alloy. In a variant, the heat-conducting material is in ceramic.

The or each thermal bridge may be a heat-conduction fluid (also known as a heat transfer fluid), such as a liquid or a gel.

The density of the heat-conducting material may be greater than or equal to 5000 kg/m³.

The thermal conductivity of the heat-conducting material may be greater than or equal to 25 W/mK.

The thermal diffusivity of the heat-conducting material may be greater than or equal to 1000.10⁸ m²/s.

The thermal effusivity of the heat-conducting material may be greater than or equal to 10,000 J/m².K.s^{1/2}.

Preferably, the or each thermal bridge mentioned above comes into contact with the thermal pad or is actually made as one piece therewith. This makes it possible, by means of conduction, to more effectively transmit to the thermal pad the cold generated by the expansion of the pressurized gas at the or each thermal bridge. The area of contact between the or each thermal bridge and the thermal pad may comprise a thermal paste. This enables the thermal transfer between the or each thermal bridge and the thermal pad at this area of contact to be improved.

The or each thermal bridge may comprise at least one hollow portion. Expansion of the pressurized gas may take place within the or each thermal bridge, notably in this hollow portion.

The gas used may be CO₂ or any other propellant gas.

The pressurized gas used may be stored in liquefied form.

Preferably, the gas used is CO₂. Indeed, the latter gas is biocompatible and the likelihood of generating a bubble on the skin is very small as compared with other gases.

The system according to the invention may comprise a regulation and control system configured such as to deliver pulses of gas into the conveying ducts.

The regulation and control system may comprise means for regulating and controlling pressure, flow rate, duration and/or frequency of the pulses of gas delivered. For example, the regulation and control system comprises at least one electrovalve and/or at least one pressure-reducing valve.

The regulation and control system may make it possible selectively to trigger the injection of the composition into the human keratin materials and/or to cool the human keratin materials. This may permit solely the injection of the composition into the human keratin materials, or solely the cooling of the human keratin materials, or simultaneous injection and cooling.

The regulation and control system may be configured such as to allow triggering of a single injection of the composition into the human keratin materials or of one sequence or of a plurality of sequences of a plurality of successive injections. This may make it possible to effect injections one by one or as a burst.

The system according to the invention may comprise a cartridge comprising:
- at least one first chamber containing and/or designed to receive the composition to be injected,
- at least one injection nozzle communicating with the or each first chamber,
the cartridge comprising a plurality of first chambers and/or the cartridge comprising at least one second chamber containing the composition to be injected and communicating with the first chamber(s) via at least one supply duct via which the first chamber(s) is (are) supplied with the composition contained in the second chamber.

In one embodiment of the invention, the cartridge comprises a first chamber designed to receive a composition to be injected, an injection nozzle communicating with the first chamber and at least one second chamber containing the composition to be injected and communicating with the first chamber via at least one supply duct via which the first chamber is supplied with the composition contained in the second chamber. When there is a nozzle with a plurality of outlet ducts, the arrangement of the outlet channels may match the form of the area to be treated, for example match the form of a wrinkle to be filled.

In a variant, the cartridge comprises a plurality of first chambers each containing a composition to be injected and at least one injection nozzle communicating with each first chamber.

In another variant, the cartridge comprises a plurality of first chambers each containing and/or designed to receive a composition to be injected, at least one injection nozzle communicating with each first chamber and at least one second chamber containing the composition to be injected and communicating with the first chambers via at least one supply duct via which the first chambers are supplied with the composition contained in the second chamber.

When there is a plurality of first chambers, the outlet channels of the corresponding nozzles may, as in the case of a single first chamber, match the form of an area to be treated, for example a wrinkle.

The cartridge offers the advantage of being ready for use and of not requiring adjustment on the part of the user prior to use. It may, on account of its geometry and dimensions, be specifically dedicated to one particular type of use. For example, the geometry and the dimensions of the first chamber and second chamber of the injection nozzle are tailored to the volume and to the nature of the composition to be injected, notably the rheology thereof, with a view to effecting a given treatment.

The second chamber may constitute a multi-dose reservoir for the composition to be injected. The second chamber may also contain the composition to be injected in a quantity sufficient to carry out a full treatment session involving a plurality of injections.

After a dose of the composition contained in the first chamber has been injected, this first chamber can be refilled automatically with the composition contained in the second chamber, with no need to replace the cartridge. This makes it possible to reduce handling operations during treatment.

Preferably, notably when the composition is pressurized in the first chamber for injection, the or each supply duct comprises a shut-off member, which is, for example, a ball. This latter may close off the supply duct during injection.

Preferably, the shut-off member is kept closed by the pressure in the first chamber during injection and opens up between two injections to allow the first chamber to fill with the composition contained in the second chamber.

The cartridge may be supplied to the user with or without the first chamber(s) being pre-filled with a single dose of the composition to be injected.

The or each first chamber may be pressurized in various ways.

Preferably, the first chamber receives the first piston connected to the push rod, the first piston sliding along the longitudinal axis of the first chamber and being driven by the push rod. The pressure exerted on this first piston during injection may be between 400 kPa and 2 MPa. Preferably, the injection nozzle forms the distal end of the cartridge. The nozzle comprises at least one outlet channel, the or each outlet channel comprising an opening through which the composition to be injected is expelled to the exterior.

Preferably, the first chamber, the second chamber, the injection nozzle and the supply duct are defined at least in part by a body formed as a single piece. Such a one-piece construction may offer enhanced strength and proves to be simpler to manipulate and to use.

The cartridge may make it possible to effect a number of injections greater than or equal to 3 or, better, greater than or equal to 10, without having to be replaced.

Preferably, the volume V₁ of the first chamber is less than that V₂ of the second chamber. This makes it possible for the first chamber to be supplied or refilled several times over with the composition contained in the second chamber so that a plurality of injections can be performed using the same cartridge. During an injection, all or some of the contents of the first chamber may be delivered to the user. In other words, the first chamber may be emptied in a single shot during injection or, in a variant, a plurality of injections are performed using the contents of the first chamber before it is refilled with product, as appropriate.

Preferably, the volume of the first chamber is greater than or equal to 50 mm³.

The first chamber may of tubular form. Its wall may converge at its distal end, notably up to the outlet channel of the injection nozzle.

Preferably, the volume of the second chamber is greater than or equal to 100 mm³.

Preferably, the cross section of the opening of the or each outlet channel of the nozzle is smaller than or equal to 0.5 mm², for example being between 0.1 and 0.2 mm².

The second chamber may of annular form and extend around the first chamber. This results in a compact construction and communication between the two chambers may be achieved simply via a radial supply duct.

Preferably, the second chamber is closed at one of its axial ends by a second piston, notably one of annular form, sliding along the longitudinal axis of the second chamber. This second piston may be pushed towards the distal end of the second chamber such as to empty the latter. Pressure may be exerted continuously, by a spring, for example, or exerted only during the filling of the first chamber.

There may be various formulations and rheologies for the composition depending on the objective pursued.

Preferably, the composition is liquid and comprises a liquid phase, a mixture of a liquid phase and of a pulverulent phase and/or a mixture of a liquid phase and of a gaseous phase.

The liquid composition may have a viscosity greater than or equal to 1 mPa.s at 25°C and atmospheric pressure.

In a variant, the composition is a pulverulent phase, such as a loose powder, or a loose powder in a gaseous phase. In this case, injection is carried out not by means of actuation of the first piston but, rather, by means of a pulse of gas that draws the composition out of the cartridge, through the one or more opening(s) in the injection nozzle.

The aforementioned shut-off member may be kept open during injection such as to supply the first chamber with the composition contained in the second chamber by Venturi effect, or may even not be present, being replaced, notably, a duct.

A quantity of the composition may be located within the first chamber, between the first piston and a gas buffer. The presence of this gas buffer makes it possible, during injection, to increase the speed at which the composition is ejected from the nozzle towards the skin, so as to allow the composition to at least partially pass through the skin and, in particular, the *stratum corneum.* The *stratum corneum* corresponds to the outermost part of the skin and acts as a barrier between an organism and the environment. The *stratum corneum* is composed of dead cells, which are flattened and rich in keratin, and are also known as corneocytes.

A gas buffer of this type may be used, for example, in the presence of a liquid composition.

The gas constituting the buffer may be air, preferably ambient air.

The composition may be designed for the treatment and/or prevention of wrinkles and comprise at least one filler, notably a biopolymer such as hyaluronic acid and/or one of the salts thereof, hydroxyapatite particles, polylactic acid, an alginate, sodium monomethyltrisilanol orthohydroxybenzoate, collagen, an acrylic polymer such as polyacrylamide, a methacrylic polymer and/or dextran microspheres.

The composition may notably comprise a liquid medium with particles in a dispersed state in the liquid medium, and may be solidified by the aggregation of said particles.

Preferably, the composition comprises at least one biopolymer, notably an alginate, and an ionic solution, notably a divalent ion solution.

The invention may make it possible to combat the cutaneous signs of ageing and notably to maintain and/or to restore the viscoelastic or biomechanical properties of the skin with a view to improving the firmness, elasticity and tone of the skin and preventing the appearance of cutaneous signs of ageing.

In the context of the present invention, "viscoelastic or biomechanical properties of the skin" is understood to mean the extendibility, tone, firmness, suppleness and/or elasticity properties of the skin.

"Cutaneous signs of ageing" is understood to mean any modifications in the external appearance of the skin due to ageing, whether chrono-biological and/or extrinsic, chiefly photo-induced ageing, such as, for example, wrinkles and fine lines, crepiness, flaccid skin, thinned skin, dull skin lacking in radiance, lack of skin elasticity and/or tone.

Preferably, the composition is a cosmetic composition but in a variant it exerts a dermatological action.

The cartridge may be arranged so as to be received in a housing of the injection system. In a variant, the cartridge is configured to be fixed removably to the injection system, notably by screw-fastening or bayonet-fixing.

The user can thus change the cartridge easily to suit his/her needs.

Preferably, the injection system comprises the thrust element that moves the push rod along the longitudinal axis of the injection system.

The system may comprise at least one actuator, notably of annular form, so as to move the second piston that closes one of the ends of the second chamber along the longitudinal axis of the injection system.

Preferably, the thrust element and/or the push rod which enable the first chamber to be emptied and the actuator which enables the second chamber to be emptied are moved along the longitudinal axis of the system towards the injection nozzle by the pressurized gas contained in the gas cartridge.

In a variant, the thrust element and/or the push rod and the actuator are moved along the longitudinal axis of the system towards the injection nozzle by other drive means, such as a spring, a motor or an electromagnet.

Preferably, the system comprises at least one elastic return member making it possible to accompany or damp the movement of the thrust element and/or the push rod and/or assisting these in returning to their initial position after injection.

The needleless injection system according to the invention may comprise a system for automatic recognition of the treated area, which is configured such as automatically to determine the location and the depth of the area to be treated when the injection system is brought close to the skin and automatically to adapt the injection parameters, notably the injection depth. This system may generate information regarding the cartridge to be used and/or the pressure to be applied using the aforementioned drive means, so as to achieve the desired penetration into the skin.

A further subject of the invention according to another of its aspects is a method for the treatment, notably cosmetic, i.e. non-therapeutic, treatment, of human keratin materials, comprising the following steps:
a) providing a needleless injection system comprising a composition to be injected and a reservoir containing a pressurized gas according to the invention as defined above,
b) placing the needleless injection system close to the human keratin materials to be treated,
c) generating cold by means of an expansion of the pressurized gas,
d) ejecting outside of the needleless injection system, notably delivering into said human keratin materials, a quantity of the composition to be injected by using the pressure of the gas, the ejection of said quantity of the composition outside of the needleless injection system being carried out as a single ejection or as one sequence or as a plurality of sequences of a plurality of successive injections,
e) cooling said human keratin materials by means of the cold generated by the expansion of the pressurized gas.

Step d) of the method according to the invention may be performed before and/or during step c). In a variant, it is performed afterwards.

Step e) of the method according to the invention may be performed before and/or during step d). In a variant, step e) is performed after step d).

The method according to the invention may comprise the following steps:
i. the intake of a quantity of the composition to be injected into a first chamber under a first piston,
ii. the raising of the first piston into the first chamber such as to draw in ambient air via an opening in an outlet channel of an injection nozzle so as to form an air buffer in the first chamber under the composition within the first chamber,
iii. the lowering of the first piston in the first chamber such as to eject the composition present within the first chamber outside of the needleless injection system through the opening in the outlet channel of the injection nozzle.

Preferably, step iii. takes place immediately or very shortly after the end of step ii., for example less than 1 second after the end of step ii.

Preferably, the human keratin materials are cooled by at least 3°C, preferably at least 5 to 10°C, relative to their initial temperature, which is, for example, between 30 and 37°C. For example, when the initial temperature of the human keratin materials to be treated is 37°C, they may be brought to a temperature below or equal to 34°C, preferably below or equal to 32-27°C.

The method according to the invention may comprise the step consisting in refilling the first chamber between two injections with a dose of composition taken from the second chamber of the cartridge.

Preferably, the composition is injected into the keratin materials to a depth greater than or equal to 10 microns. This depth may not be enough to pass through the dermis and, for example, be less than or equal to 2 mm or even 1 mm. The invention makes it possible notably to inject the composition into the epidermis and/or dermis. With a view to influencing the injection depth, the pressure in the first chamber and the speed at which the composition is ejected from the nozzle towards the skin may be altered.

The method according to the invention may make it possible to prevent, treat and/or alleviate a disorder or defect in human keratin materials, preferably an aesthetic, i.e. non-pathological, disorder or defect of the skin.

The method according to the invention may further comprise a step consisting in observing an alleviation or even a disappearance of the aesthetic disorder in question.

A further subject of the invention according to another of its aspects is the cosmetic, i.e. non-therapeutic, use of a needleless injection system according to the invention, as defined above, in order to improve the appearance, notably the aesthetic appearance, of the skin.

In particular, the needleless injection system according to the invention may be used to prevent, treat and/or alleviate an aesthetic, i.e. non-pathological, defect or disorder of an area of the skin, such as a relief and/or a fold in an area of the skin, notably a fine line, wrinkle and/or scar.

### Composition

The composition to be injected according to the invention is preferably liquid, at least at the moment when it is injected.

The composition may be a liquid phase, a mixture of a liquid phase and of a pulverulent phase, a mixture of a liquid phase and of a gaseous phase or of a liquid, a pulverulent and a gaseous phase.

The composition may have a viscosity greater than or equal to 1 mPa.s at the moment when it is injected.

The viscosity is measured at 25°C under 1 atm, using a Rheomat 180 viscometer fitted with a measuring bob MK-R-1, 2 or 3 according to the viscosity range and with the corresponding measuring tube MB-R-1, 2 or 3, at a rotational speed of 200 min⁻¹, the measurement being taken after 10 minutes of rotation (at the end of which time it is noted that the viscosity and the rotational speed of the measuring bob have stabilized).

The composition to be injected according to the invention may further be a pulverulent phase, such as a loose powder, or a loose powder in a gaseous phase.

The composition according to the invention may include a fatty phase. The fatty phase may notably comprise oils, waxes or pasty fatty substances. "Pasty fatty substance" is understood to mean a viscous product containing a liquid fraction and a solid fraction. It is understood to mean, notably, fatty substances having a melting point ranging from 20 to 55°C and/or a viscosity at 40°C ranging from 0.1 to 40 Pa.s (1 to 400 poise) measured with a Contraves TV or Rheomat 180.

The composition according to the invention may comprise an aqueous phase.

Preferably, the composition to be injected is sterile and biocompatible.

The composition to be injected may be a cosmetic composition, in which case it comprises a physiologically acceptable medium, that is to say a non-toxic medium that can be injected into the skin of human beings.

The composition to be injected may furthermore be a dermatological composition.

### Composition for treating and/or preventing a relief and/or a fold in an area of skin

### Fillers

As mentioned above, the composition according to the invention may comprise at least one filler.

The composition containing at least one filler may or may not exhibit the same rheology before and after injection into the skin. In particular, the composition may be more fluid at the moment of injection, and may solidify or become more viscous in situ.

Preferably, the filler comprises at least one biopolymer.

Owing to the mechanism of action of biopolymers and because, by virtue of the invention, they are injected into the skin in quantities and to depths that cannot be achieved by the use of topical compositions, lasting and visible effects may be obtained.

The filler may be resorbable or semi-resorbable. "Resorbable" denotes a filler that can dissolve completely within the skin in 3 to 6 months and "semi-resorbable" denotes a filler that can dissolve completely within the skin in 6 to 24 months. These time scales correspond to the time for which the action of the filler lasts. In a variant, the filler is non-resorbable.

The composition according to the invention may comprise at least one filler such as hyaluronic acid and/or one of the derivatives thereof and/or one of the salts thereof.

The hyaluronic acid is preferably crosslinked. Crosslinking makes it possible to form a rigid, elastic, crosslinked gel that hydrates and swells in water. Crosslinked hyaluronic acid is used for its filling properties, notably with regard to fine lines and/or wrinkles in the skin, because of the stiffness of the gel and also its highly viscoelastic properties and high hydrating ability.

In a variant, the hyaluronic acid is not crosslinked.

Hyaluronic acid may, notably, be used in a mixture with lidocaine and/or dextran microspheres.

The composition to be injected may be a solution from the BELOTERO^{®} range marketed by Merz, notably the solution BELOTERO^{®} Soft.

The composition according to the invention may comprise hydroxyapatite particles, notably calcium hydroxyapatite particles, polylactic acid, an alginate, sodium monomethyltrisilanol orthohydroxybenzoate, collagen, an acrylic polymer such as polyacrylamide, a methacrylic polymer, and any mixture of the above compounds.

### Variable-rheology agents

The composition to be injected according to the invention may be a composition containing at least one variable-rheology agent. The viscosity of this composition therefore increases between the moment at which the composition is prepared and a moment after injection, after it has reached its destination in the skin.

The increase in viscosity may occur through gelling or through any other physical phenomenon that causes particles or molecules of the composition to agglomerate, for example through the effect of electrostatic or coalescing forces, leading to the creation of a kind of net or mesh.

The aggregation process may be triggered by heat, when the temperature of the composition rises above a certain value, which is preferably chosen to be close to 37°C. Thus, the composition when injected may have a viscosity lower than that which it subsequently has in situ in the skin at the point of injection. This makes it easier to inject without adversely affecting its performance in terms of filling skin defects.

Prior to injection, the viscosity of the composition is, for example, greater than or equal to 1 mPa.s at 25°C at atmospheric pressure.

The composition may be a liquid composition containing particles dispersed in a liquid medium, it being possible for said composition to solidify by means of the aggregation of the particles.

The composition may comprise at least one biopolymer, preferably an alginate, and a solution of ions, particularly a solution of divalent ions. The viscosity of the biopolymer advantageously increases in the skin because of the gelling of the biopolymer until the latter becomes solid. Owing to the mechanism whereby biopolymers aggregate and are injected into the skin in quantities and to depths that cannot be achieved by the use of topical compositions, lasting and visible effects may be obtained.

One of the biopolymers considered according to the invention may be the Novabel^{®} alginate by Merz, which can be rebsorbed in 3 to 6 months.

In a preferred embodiment, the composition comprises a sodium alginate and a calcium salt solution, preferably a calcium carbonate CaCO₃ solution. The improved reactivity of alginates with calcium ions has been described in the paper by B. Larsen et al. entitled "Caractérisation rhéologique d'un système de gel d'alginate injectable" ["Rheological characterization of an injectable alginate gel system"], BMC Biotechnology 2015, pages 15-29. A solution of active compounds, such as a calcium carbonate solution, makes it possible, by ionic energy transfer, to generate in situ a precipitate that forms a film or a scaffold within the layers of the skin.

One particularly suitable sodium alginate is derived from brown algae and may be an injectable FMC^{®} polymer powder dissolved in water. Such a compound is a hydrophilic colloid, or hydrocolloid, defined as a colloid system in which the colloid particles are hydrophilic polymers dispersed in water.

The biopolymer and the ion solution may be mixed before being packaged in the system. In a variant, the biopolymer and the ion solution are contained in different reservoirs and injected separately and one after the other. The aggregation of the composition is advantageously triggered by heat after injection into the skin.

The composition may be resorbable, semi-resorbable or non-resorbable.

### Additives

The composition according to the invention may comprise at least one conventional cosmetic ingredient other than a filler, which may, notably, be selected from antioxidants, sunscreens, vitamins, moisturisers, anti-wrinkle active ingredients, emollients, hydrophilic or lipophilic active ingredients, agents combating free radicals, and mixtures thereof.

In the long term, injecting the composition into human keratin materials may stimulate the user's own production of collagen by collagen-producing cells. Lasting effects may thus be observed, notably the filling of fine lines and/or wrinkles in the skin.

The invention may be better understood from reading the following detailed description of non-limiting exemplary embodiments thereof and from studying the appended drawing, in which:
- Figure 1 schematically shows a perspective view of an example of a needleless injection system according to the invention,

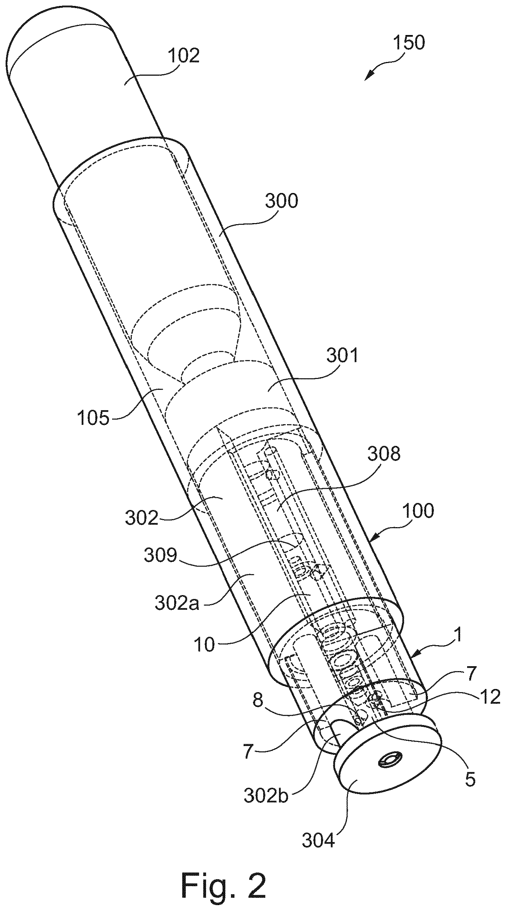

- Figure 2 is a view similar to Figure 1,
- Figure 3 is a longitudinal section on plane III in Figure 1,
- Figure 3A shows a detail from Figure 3, without the valve,
- Figure 4 schematically shows an example of an injection system according to the invention,
- Figure 5 schematically shows a perspective view of an example of a cartridge comprising a composition to be injected according to the invention,
- Figure 6 is a longitudinal section on plane A in Figure 5,
- Figure 7 is a view similar to figure 6, without the push rod, and
- Figure 8 shows an embodiment detail of the injection system.

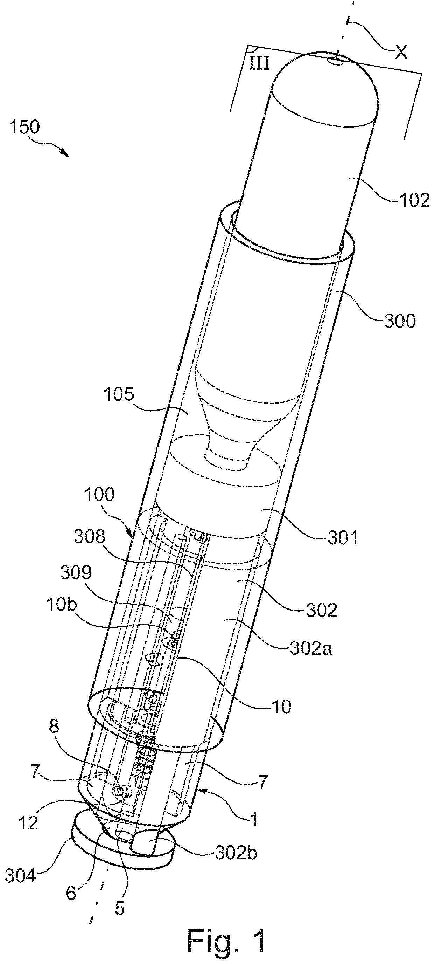

Figure 1 shows an example of a needleless injection system 150 according to the invention.

This system 150 extends generally along an axis X of elongation. It comprises an injection device 100 and a cartridge 1 comprising the composition to be injected.

The cartridge 1 comprises a first chamber 5 designed to receive the composition to be injected, an injection nozzle 6 communicating with the first chamber 5 and at least one second chamber 7 containing the composition to be injected and communicating with the first chamber 5 via at least one supply duct 8 via which the first chamber 5 is supplied with the composition contained in the second chamber 7.

The or each supply duct 8 comprises a shut-off member 12, which is, for example, a ball. The latter may close off the supply duct 8 during injection.

The system 150 preferably comprises a hand piece 300 made from a thermoplastic material, notably a polycarbonate-based material.

The hand piece 300 may be given various forms.

Preferably, the hand piece 300 has an elongate form to make it easier to hold in the hand and to manipulate.

The system 150 comprises a reservoir containing a pressurized gas. The reservoir may be a gas cartridge 102.

If desired, a compact injection system 150 may be used, incorporating the gas cartridge 102 into the hand piece 300, as illustrated in Figure 1. The handpiece may then comprise a housing 105 for receiving the cartridge 102.

The cartridge 102 may be removable and replaceable. It may or may not be refillable. Preferably, the gas cartridge 102 contains liquefied CO₂.

Preferably the cartridge 102 is compact. It has, for example, a gas mass of less than 50 g, for example 12 g or 16 g, a length of less than 10 cm and a diameter of less then 2 cm.

The cartridge 102 may contain a quantity of gas sufficient to perform a number of injections in excess of or equal to 100. Such autonomy may allow treatment of all facial wrinkles.

The pressure within the cartridge 102 is, for example, greater than 25 bar, and, better, greater than 50 bar.

The system 150 may comprise at least one thermal bridge 302 within which the pressurized gas expands. This thermal bridge 302 is configured such as to convey the cold generated by the expansion of the pressurized gas to a thermal pad 304 configured such as to come into contact with human keratin materials. This thermal pad 304 may be of annular form, injection being carried out through the pad.

The or each thermal bridge 302 may comprise a hollow body 302a extending via a rod 302b coming into contact with the thermal pad 304, as illustrated in Figure 1. In a variant, the element 302b is a ring.

Preferably, the or each hollow body 302a is located in the hand piece 300.

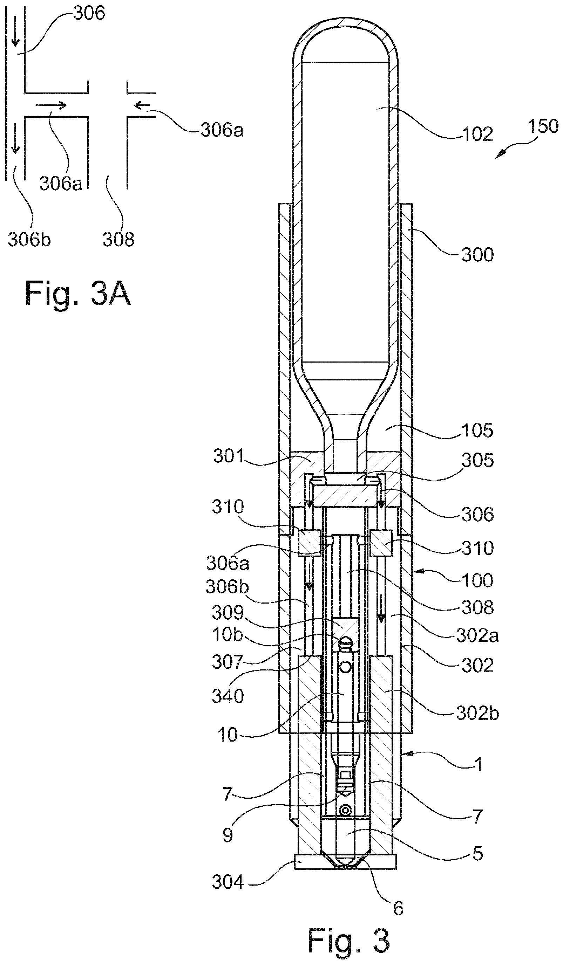

The system 150 may comprise a connection and distribution interface 301 comprising a connector 305 making it possible to place the outlet from the gas cartridge 102 in communication with a plurality of conveying ducts 306 arranged in parallel with the longitudinal axis of the injection system 150, as illustrated in Figure 3.

Preferably, the connection and distribution interface 301 is located in the hand piece 300.

The gas cartridge 102 is configured such as to connect removably to the inlet of the connection and distribution interface 301, notably by means of screw-fastening or bayonet-fixing. Preferably, this connection is achieved rapidly and securely, without tools. The inlet of the interface 301 may comprise an O-ring that guarantees the leaktightness of the connection. When the cartridge 102 is closed by a film that can be pierced, the inlet of the interface 301 may comprise a needle for piercing this film.

As illustrated in Figure 3, the or each conveying duct 306 may comprise a branching 306a that makes it possible to convey a portion at least of the gas contained in the cartridge 102 towards an actuation chamber 308. This chamber 308 receives a thrust element 309 connected to a head 10b of a push rod 10. Pressurizing of the chamber 308 enables injection to be performed by actuating the thrust element 309, the push rod 10 and a first piston 9 located in the extension of the rod 10.

The thrust element 309 and/or the push rod 10 may comprise at least one elastic return member (not shown) configured such as to assist in their movement in the course of injection or, alternatively, to damp their movement or to assist the thrust element 309 and/or the push rod 10 in returning to their initial position after injection.

The or each conveying duct 306 may also comprise a duct 306b received within the hollow body 302a in order to convey a portion at least of the gas contained in the cartridge 102 towards a discharge area 307 located within the thermal pad 302. This area 307 may be located opposite the rod or ring 302b.

The duct 306b comprises an opening in the discharge area. This makes it possible to promote a high-speed gaseous stream at the opening and hence more significant cooling generated by the expansion of the gas.

The discharge area 307 has a surface 340 configured such as to capture the cold generated by the expansion of the pressurized gas at the opening of the duct 306b. This surface 340 may be located close to and/or opposite the opening of the duct 306b.

The injection device 100 comprises a propulsion mechanism that may comprise the actuation chamber 308, the thrust element 309, the push rod 10 and the first piston 9.

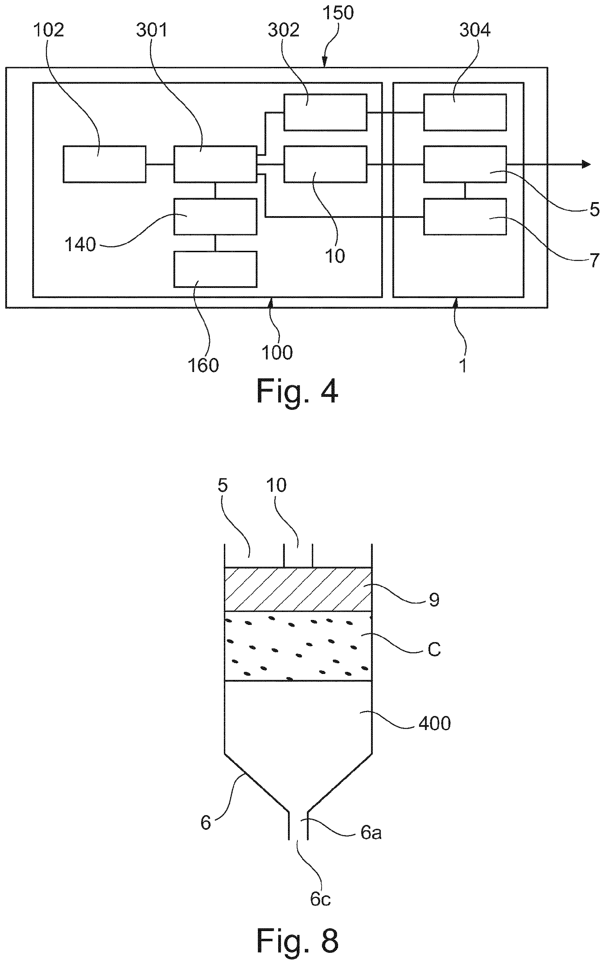

As illustrated in Figure 4, the injection system 150 may comprise an injection device 100 and a cartridge 1 containing the composition to be injected. The injection device 100 may comprise a regulation and control system 140 comprising, for example, at least one valve and/or at least one pressure-reducing valve. This control system 140 may make it possible to control the connection and distribution interface 301 and, notably, to allow the delivery of pulses of gas into the conveying ducts 306. The control system 140 may make it possible to regulate parameters such as pressure, flow rate, duration and frequency of the pulses of gas.

The regulation and control system 140 may comprise a manually or electrically controlled valve 310 allowing a supply to the branching 306a of the propulsion mechanism and a supply to the duct 306b of the discharge system. This makes it possible to cool and to inject concomitantly. In a variant, the branching 306a may be supplied separately from the duct 306b. This makes it possible to obtain a configuration that allows cooling only or a configuration that allows injection only.

The valve 310 may be controlled by user action on a control member, such as a push button (not shown), for example, it being possible for such a member to be located on the hand piece 300.

In the configuration that allows cooling only, the valve 310 is configured such as to supply solely the duct 306b of the discharge system with a portion at least of the gas contained in the cartridge 102. The expansion of a portion at least of the gas contained in the cartridge 102 then takes place right along the duct 306b up to the discharge area 307. This expansion makes it possible to generate cold, notably in the discharge area 307. This cold is transmitted to the thermal bridge 302, notably to the hollow body 302a receiving the duct 306b and to the area of the rod or ring 302b opposite the discharge area 307. The thermal bridge 302 conveys the cold to the thermal pad 304. This configuration may make it possible to cool human keratin materials before and/or after injection.

In the configuration that allows cooling only, the valve 310 is configured such as to supply solely the branching 306a of the propulsion mechanism with a portion at least of the gas contained in the cartridge 102.

In the configuration that allows cooling and injection concomitantly, the valve 310 is configured such as simultaneously to supply the duct 306b and the branching 306a with a portion at least of the gas contained in the cartridge 102. The flow rate and/or the pressure of gas in the duct 306b and the branching 306a may or may not be identical.

In a variant, the regulation and control system 104 does not comprise a valve 310, as illustrated in Figure 3A. The gas originating from the cartridge 102 is then directed into both the branching 306a and the duct 306b. The flow rate and/or the pressure of gas exiting the cartridge 102 are chosen such that the flow rate and/or the pressure of gas in the branching 306a are sufficient to actuate the propulsion mechanism. This enables cooling and injection to take place concomitantly, without control by a valve 310.

Injection of the composition into the human keratin materials and/or the cooling of the human keratin materials may be controlled by user action on a control member, such as a push button (not shown), for example, it being possible for such a member to be located on the hand piece 300.

The regulation and control system 140 may be configured such that user action on the control member triggers a single injection or, in a variant, one or a plurality of sequences of a plurality of successive injections. Injections may thus be carried out one by one or as a burst. The regulation and control system 140 may make it possible to regulate parameters such as the number and frequency of injections in the course of the sequence, the duration of the sequence and, if appropriate, the number of sequences and the period of time between each sequence.

Similarly, the regulation and control system 140 may be configured such that user action on the control member triggers a single expansion of pressurized gas in order to generate cold or, in a variant, one or a plurality of sequences of a plurality of successive expansions.

The control system 140 may be electrically connected to a power supply 160. The role of the power supply 160 is to provide electrical energy to the control system in order that the latter can function. The power supply 160 may be a fuel cell or a battery, notably a rechargeable fuel cell or battery, a mains power supply, or any other source of electrical power.

The expansion of the pressurized gas contained in the cartridge 102 may allow actuation of the push rod 10 in order forcibly to expel the composition contained in the first chamber 5 into the keratin materials and, where appropriate, pushing of the reserve of composition contained in the second chamber 7 towards the first chamber 5 in order to refill the latter. The injection device 100 may comprise the push rod 10. In a variant, the latter is present in the cartridge 1.

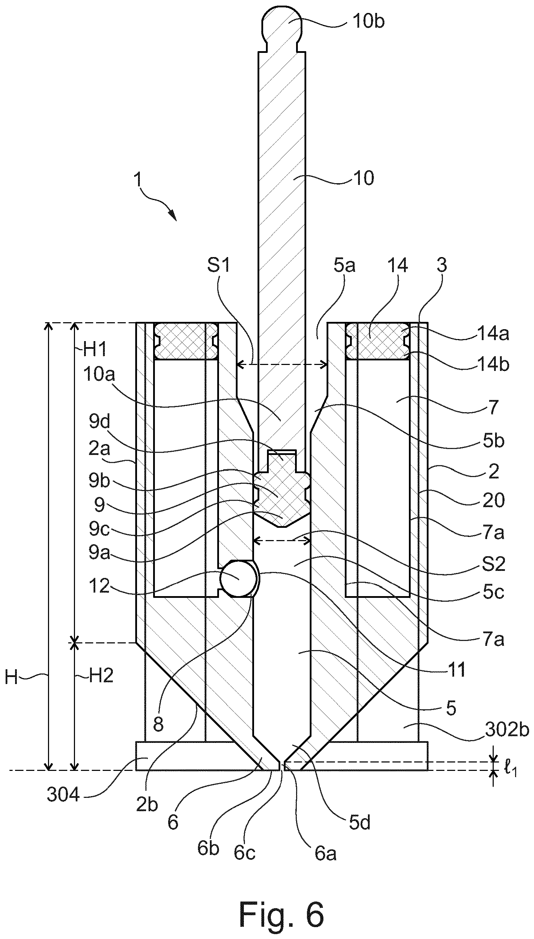

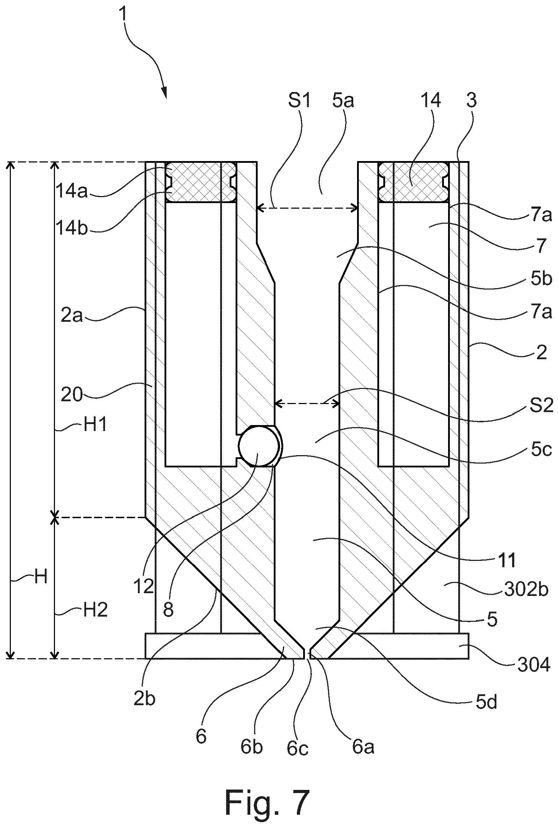

Expansion of the pressurized gas contained in the cartridge 102 may also make it possible to generate cold within the thermal bridge 302, this cold being transmitted by conduction to the thermal pad 304. The cartridge 1 may comprise a portion at least of the or of each thermal bridge 302 and, notably, a portion at least of the rod or ring 302b of the or of each thermal bridge 302, as illustrated in Figures 6 and 7.

The connection and distribution interface 301, the or each conveying duct 306, the or each thermal bridge 302 and the thermal pad 304 at least are preferably made from a material exhibiting good heat-conducting properties.

The injection system 150 may comprise a removable, replaceable cartridge comprising the composition to be injected and located in the extension of the hand piece 300. In a variant, the cartridge is not removable or replaceable.

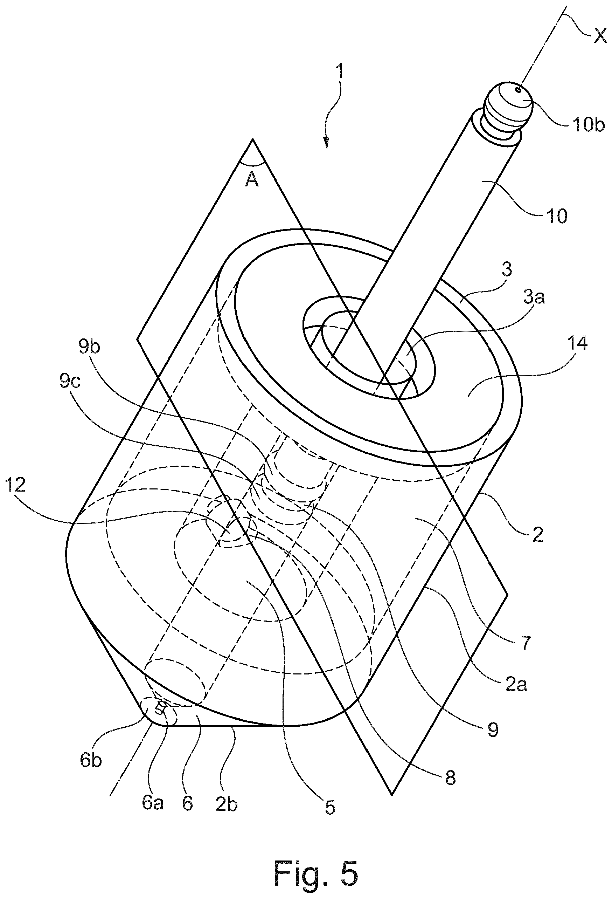

Figure 5 shows an example of a cartridge 1 that extends generally along an axis X of elongation. To simplify Figure 5, a portion at least of the rod or ring 302b of the or each thermal bridge 302 has not been shown.

The cartridge 1 is configured to come into contact with and, notably, to be inserted into the injection device 100. The cartridge 1 may be fixed to the injection device 100 by any means.

The cartridge 1 comprises a body 2 that has a cylindrical portion 2a with an opening 3a at an end 3.

The cylindrical portion 2a extends at the opposite end from the end 3 in the form of a frustoconical portion 2b forming the injection nozzle 6. The cartridge 1 is configured such as to be mounted on an injection device, which is not shown in Figure 5. The injection nozzle 6 has an outlet channel 6a and an end face 6b to be positioned facing or in contact with the human keratin materials to be treated.

The cartridge 1 comprises some at least of the rods or rings 302b of the thermal bridges 302 (shown in transparent form in Figure 6 in order to make it easier to understand the figure), these coming into contact with the thermal pad 304.

The cartridge 1 also comprises the first chamber 5 designed to receive the composition to be injected. The channel 6a of the injection nozzle 6 communicates with this first chamber 5.

The second chamber 7, containing the reserve of composition, communicates with the first chamber 5 via at least the supply duct 8.

In the example illustrated, the first chamber 5 occupies a central position in the cartridge 1 and has a cylindrical first portion 5a, of cross section S1, and a cylindrical second portion 5c, of cross section S2 smaller than the cross section S1. The first and second portions 5a and 5c are connected by an intermediate portion 5b that converges towards the distal end of the cartridge 1. The first chamber 5 has a distal portion 5d of which the wall converges towards the distal end of the cartridge 1, this portion 5d forming the end wall of the first chamber 5. The latter may, as illustrated, have a circular cross section over its entire length.

The volume of the first chamber 5 is, for example, greater than or equal to 50 mm³.

The outlet channel 6a may have a constant cross section, for example a circular cross section, along its entire length.

In a variant, the injection nozzle 6 comprises a plurality of outlet channels 6a, each being connected to the end wall 5d of the first chamber 5 and each opening out at the end of the nozzle via a corresponding opening 6c.

The opening 6c of the or each outlet channel 6a of the injection nozzle 6 may be covered before first use by a removable film (not shown) that adheres to the end of the injection nozzle 6. This film ensures that the composition contained in the cartridge 1 is preserved and remains sterile.

The cross section of the opening 6c of the or each channel 6a of the nozzle 6 is, for example, smaller than or equal to 0.5 mm², in particular between 0.1 and 0.2 mm².

The first chamber 5 receives the first piston 9 of which the distal end 9a has a conical form that complements that of the end wall 5d of the first chamber 5. This first piston 9 may be made from an elastomeric material.

The first piston 9 presses in a sealed manner against the internal surface of the portion 5c of the first chamber 5 and may have one or a plurality of annular sealing lips, for example two lips 9b and 9c as illustrated.

The cartridge 1 may comprise the push rod 10 of which the distal end 10a is fixed by clip-fastening, screw-fastening, friction and/or any other means to the first piston 9. At its proximal end the rod 10 comprises the head 10b configured such as to collaborate with a drive system of the injection device.

The cartridge 1 is arranged in such a way that the first piston 9 slides within the first chamber 5 along the longitudinal axis thereof, driven by the push rod 10.

In a variant, the cartridge 1 does not comprise a first piston 9 or push rod 10, as illustrated in Figure 7. The first piston 9 may then be fixed to the distal end 10a of the push rod 10. The opening of the first chamber 5 at the proximal end 3 of the cartridge 1 may be covered prior to first use by a removable film (not shown) that adheres to the proximal end 3 of the cartridge 1. This film ensures that the composition contained in the cartridge 1 is preserved and remains sterile.

The second chamber 7 may be of annular form and extend around the first chamber 5, as illustrated. Preferably, the volume of the second chamber 7 is greater than that of the first chamber 5. The volume of the second chamber 7 is, for example, greater than or equal to 100 mm³.

The second chamber 7 communicates with the first chamber 5 via the supply duct 8. The latter may be perpendicular to the axis of the chamber 5, as illustrated, or angled upwards or downwards.

This duct 8 is equipped with a valve 11 that opens in the direction of flow of the composition from the second chamber 7 to the first chamber 5. This valve has no spring for returning the shut-off member 12, in this instance constituted by a ball, to rest.

In a variant, the second chamber 7 communicates with the first chamber 5 via a plurality of supply ducts 8. This allows better transfer of the composition from the second chamber 7 to the first chamber 5, notably faster transfer.

The second chamber 7 is closed at one of its ends by a second piston 14 that in the example considered is of annular form. This second piston 14 presses in a sealed manner against the interior wall 7a of the second chamber 7. It may have one or a plurality of sealing lips 14a and 14b.

The second piston 14 may be driven by any means in the direction of an emptying of the second chamber 7.

The first chamber 5, the second chamber 7, the injection nozzle 6 and the supply duct 8 may be formed within a one-piece component. In a variant, they are formed by an assembly of a plurality of components.

The cartridge 1 may be made from a thermoplastic material, notably a polycarbonate-based material. The elements of the cartridge 1 that are in contact with the composition to be injected, notably the wall of the first chamber 5, of the second chamber 7, of the supply duct 8 and of the outlet channel 6a, may be covered with glass, notably silicone glass and/or depyrogenated glass.

In a variant, if the viscosity of the composition so permits, the radially exterior wall 20 of the cartridge 1 is made from a deformable flexible material. This then allows the user to supply the first chamber 5 with the composition contained in the second chamber 7 by applying pressure, for example finger pressure, to the wall 20.

In another variant, the cartridge comprises a visual indicator of the fill level of the composition contained in the second chamber 7, for example a transparent window made in the wall 20.

The cartridge may be specifically dedicated to one application in particular, notably to the treatment and/or prevention of folds on the surface of the skin, for example folds in the labionasal area (also referred to as smile lines), in the crow's feet area, in the area between the eyebrows and/or in the forehead area. The cartridge may be dedicated to use on fine lines, surface wrinkles and/or deep wrinkles.

The nature of the active compound present in the composition to be injected, and the volumes and dimensions of the first chamber and second chamber and of the injection nozzle are tailored to suit the target application and the physical/chemical properties of the composition, such as particle size distribution when the composition is a loose powder or viscosity when the composition is liquid.

In a variant, the cartridge 1 comprises a plurality of second chambers. Each second chamber communicates with the first chamber via at least one supply duct. Each second chamber may be of tubular form and be arranged on the exterior of the first chamber, being closed at its proximal end by a corresponding second piston. These second chambers may all contain the same composition to be injected. In a variant, the second chambers contain different compositions. This makes it possible for one and the same cartridge 1 to be used in a plurality of applications. If appropriate, at least one of the second chambers contains a cleaning product instead of a composition to be injected. This allows the cartridge 1, and particularly the injection nozzle 6, to be cleaned between two injections of different compositions. The cleaning solution may be selected from one of the solvents of the composition to be injected in order to be compatible therewith and include isodecane, a volatile silicone or else alcohol or water.

The cartridge may or may not be refillable.

As illustrated in Figure 8, notably but not exclusively when the composition C to be injected into the human keratin materials is liquid, the injection system 150 may comprise a quantity of composition C located within the first chamber 5, between the first piston 9 and a gas buffer 400.

Preferably, the gas constituting the buffer 400 is ambient air.

The air buffer 400 may be obtained by means of the first piston 9 rising within the first chamber 5, which causes a quantity of ambient air to be drawn through the opening 6c in the or each outlet channel 6a of the injection nozzle 6, this quantity of ambient air drawn through forming the air buffer 400.

Preferably, the height of the gas buffer 400 is greater than or equal to half the height of the first piston 5.

The cartridge may comprise means for heating the composition to be injected or the area of human keratin materials to be treated.

The cross section and the radius of curvature of the end face 6b of the injection nozzle 6 are chosen, as appropriate, according to the type of keratin materials treated, the face 6b then being applied directly into contact with the keratin materials treated. The radius of curvature may be chosen such as to substantially match the curvature of the treated area. This makes it possible to facilitate the application of the injection system to certain body surfaces. For example, the face 6b has a shape that is outwardly concave.

A plurality of cartridges 1 having nozzles of different forms may be offered to the user for mounting on the injection device. This makes it possible to select a cartridge 1 that has the injection nozzle that is best suited, in terms of form, to the area of keratin materials to be treated.

In a variant that has not been illustrated, the cartridge comprises a plurality of first chambers 5 equipped with their respective pistons, such as to allow a plurality of injections in parallel, simultaneously or sequentially. These first chambers may be supplied by one and the same second chamber or by respective second chambers. All the above description relating to an exemplary embodiment comprising only a single first chamber 5 also applies to a variant comprising a plurality of first chambers.

The injection system 150 may comprise an actuator (not shown), notably of annular form, configured such as to cause the second piston 14 to slide within the second chamber 7 along the longitudinal axis of the injection device 150. This makes it possible to move the reserve of composition contained in the second chamber 7 towards the first chamber 5 so as to refill the latter.

The gas cartridge 102 may make it possible to drive the actuator. In a variant, the actuator is actuated by an electromagnet or a spring, the compression of which may be manual or motorized, or by any other means.

In one variant, the cartridge 1 may comprise a plurality of second chambers 7 arranged all around the first chamber 5 and each closed by a second piston at the proximal end thereof. The cartridge 1 may be mounted on the injection device 100 with the ability to rotate. The rotating of the cartridge 1 on the injection device allows the actuator to be aligned with one of the second chambers in a carousel-type arrangement. This rotation may be performed manually or may be motorized. This means the user can select that one of the second chambers 7 from which he or she wishes to supply the first chamber 5.

In another variant, the device comprises a plurality of actuators.

The supply to the first chamber 5 from the reserve of composition contained in the second chamber 7 may likewise as appropriate be carried out by means of an element that the user can actuate.

The injection system 150 may be offered to the user with one or a plurality of cartridges 1 containing one or a plurality of compositions to be injected, for example within common packaging, it being possible for these cartridges each to have a nozzle specific to the type of treatment to be performed.

The injection system 150 may constitute a portable, autonomous system.

The injection system 150 may, where appropriate, comprise a system for recognizing the cartridge 1, for example by means of an electromechanical sensor, electrical contacts, or an electronic chip. For example, the cartridge 1 comprises an RFID chip read by the injection device 100. The cartridge 1 may thus be automatically recognized by the injection device 100. The fact that the device is aware that the cartridge is in place may allow operating parameters of the device, for example pressure, duration, flow rate and/or frequency of the pulses of gas leaving the gas cartridge 102, to be adapted automatically.

In a variant, the operation and/or setting of the injection system 150 is remotely controlled, for example with a smartphone. The injection system 150 then comprises a wireless communication interface.

The invention is not limited to the examples that have just been described. For example, in one variant, the propulsion mechanism of the injection system has no push rod or first piston for pressurizing the contents of the first chamber.

For example, the composition is pulverulent and injected from the first chamber by means of a gas jet. In this case, the supply to the first chamber is carried out, for example, by Venturi effect. The speed of the entrainment gas at the outlet of the nozzle is chosen such as to be sufficient to cause the composition to at least partially penetrate the skin.

The term "comprising" should be understood in its commonly accepted meaning, namely as being synonymous with "comprising at least one", unless specified to the contrary.

## Claims

1. Needleless injection system (150) for injecting a composition, the system (150) comprising and/or designed to receive a reservoir (102) containing a pressurized gas, notably stored in gaseous and/or liquefied form, the system (150) comprising conveying ducts (306; 306a; 306b), enabling the gas to be directed,
first, towards the human keratin materials in order to cool said materials by means of the cold generated by an expansion of the pressurized gas and/or towards a surface (340) configured such as to capture the cold generated by an expansion of the pressurized gas and to transmit it towards the human keratin materials in order to cool the human keratin materials,
and, second, towards a mechanism (308; 309; 10; 9) for propelling the composition into the human keratin materials using the gas pressure.

2. System according to Claim 1, comprising a hand piece (300) comprising and/or designed to receive the reservoir (102) containing the pressurized gas.

3. System according to Claim 1 or 2, comprising a thermal pad (304) cooled by the cold generated by the expansion of the pressurized gas and configured such as to come lean on the human keratin materials.

4. System according to Claim 3, the thermal pad (304) being of annular form and injection being carried out through said pad.

5. System according to Claim 3 or 4, comprising at least one thermal bridge (302) within or in contact with which the pressurized gas expands, the or each thermal bridge (302) being configured such as to convey the cold generated by the expansion of the pressurized gas to the thermal pad (304).

6. System according to any one of Claims 1 to 5, the composition being a pulverulent phase, such as a loose powder, or a loose powder in a gaseous phase.

7. System according to any one of Claims 1 to 5, the composition being liquid and comprising a liquid phase, a mixture of a liquid phase and of a pulverulent phase, a mixture of a liquid phase and of a gaseous phase, or a mixture of a liquid, a pulverulent and a gaseous phase, the composition having preferably, at least at the moment of its injection, a viscosity greater than or equal to 1 mPa.s at 25°C and atmospheric pressure.

8. System according to Claim 7, the composition comprising a dispersion of particles in a liquid medium, it being possible for said composition to solidify via the aggregation of said particles, the composition comprising preferably at least one biopolymer, notably an alginate, and an ionic solution, notably a divalent ion solution.

9. System according to any one of the preceding claims, a quantity of the composition being located within a first chamber (5) between a first piston (9) and a gas buffer (400).

10. System according to any one of the preceding claims, the composition comprising at least one filler, notably a biopolymer such as hyaluronic acid and/or one of the salts thereof, hydroxyapatite particles, polylactic acid, an alginate, sodium monomethyltrisilanol orthohydroxybenzoate, collagen, an acrylic polymer such as polyacrylamide, a methacrylic polymer and/or dextran microspheres.

11. System according to any one of the preceding claims, comprising a regulation and control system (140) configured such as to deliver pulses of gas into the conveying ducts (306; 306a; 306b), the regulation and control system (140) comprising means for regulating and controlling the pressure, flow rate, duration and/or frequency of the pulses of gas delivered, the regulation and control system (140) preferably making it possible selectively to trigger the injection of the composition into the human keratin materials and/or to cool the human keratin materials and/or the regulation and control system (140) preferably being configured such as to allow triggering of a single injection of the composition into the human keratin materials or of one sequence or of a plurality of sequences of a plurality of successive injections.

12. Method for the non-therapeutic, cosmetic treatment of human keratin materials, comprising the following steps:
a) providing a needleless injection system (150) comprising a composition to be injected and a reservoir (102) containing a pressurized gas, according to any one of Claims 1 to 11,
b) placing the needleless injection system (150) close to the human keratin materials to be treated,
c) generating cold by means of an expansion of the pressurized gas,
d) ejecting outside of the needleless injection system (150), notably delivering into said human keratin materials, a quantity of the composition to be injected by using the pressure of the gas, the ejection of said quantity of the composition outside of the needleless injection system (150) being carried out as a single ejection or as one sequence or as a plurality of sequences of a plurality of successive injections,
e) cooling said human keratin materials by means of the cold generated by the expansion of the pressurized gas.

13. Method according to Claim 12, step e) being performed before and/or during step d), or step e) being performed after step d).

14. Method as claimed in Claim 12 or 13, comprising the following steps:
i. the intake of a quantity of the composition to be injected into a first chamber (5) under a first piston (9),
ii. the raising of the first piston (9) into the first chamber (5) such as to draw in ambient air via an opening (6c) in an outlet channel (6a) of an injection nozzle (6) so as to form an air buffer (400) in the first chamber (5) under the composition present within the first chamber (5),
iii. the lowering of the first piston (9) in the first chamber (5) such as to eject the composition present within the first chamber (5) outside of the needleless injection system (150) through the opening (6c) in the outlet channel (6a) of the injection nozzle (6).

15. Method according to any one of Claims 12 to 14, the human keratin materials being cooled by at least 3°C, preferably at least 5 to 10°C, relative to their initial temperature.

## Patentansprüche

1. Nadelloses Injektionssystem (150) zum Injizieren einer Zusammensetzung, wobei das System (150) ein Reservoir (102), das ein druckbeaufschlagtes Gas enthält, insbesondere in gasförmiger und/oder verflüssigter Form gelagert, umfasst und/oder zur Aufnahme davon gestaltet ist, wobei das System (150) Beförderungskanäle (306; 306a; 306b), die Leiten des Gases ermöglichen, umfasst, erstens zu den menschlichen Keratinmaterialien, um die Materialien mithilfe der durch eine Expansion des druckbeaufschlagten Gases erzeugten Kälte zu kühlen, und/oder zu einer Oberfläche (340), die dafür gestaltet ist, die durch eine Expansion des druckbeaufschlagten Gases erzeugte Kälte aufzunehmen und sie zu den menschlichen Keratinmaterialien zu übertragen, um die menschlichen Keratinmaterialien zu kühlen,
und zweitens zu einem Mechanismus (308; 309; 10; 9) zum Treiben der Zusammensetzung in die menschlichen Keratinmaterialien unter Verwendung des Gasdrucks.

2. System gemäß Anspruch 1, umfassend ein Handstück (300), das das Reservoir (102), das das druckbeaufschlagte Gas enthält, umfasst und/oder zur Aufnahme davon gestaltet ist.

3. System gemäß Anspruch 1 oder 2, umfassend ein Wärmeleitkissen (304), das durch die durch die Expansion des druckbeaufschlagten Gases erzeugten Kälte gekühlt wird und zum Anliegen an den menschlichen Keratinmaterialien gestaltet ist.

4. System gemäß Anspruch 3, wobei das Wärmeleitkissen (304) ringförmig ist und Injektion durch das Kissen durchgeführt wird.

5. System gemäß Anspruch 3 oder 4, umfassend wenigstens eine Wärmebrücke (302) innerhalb derer oder in Kontakt mit der das druckbeaufschlagte Gas expandiert, wobei die oder jede Wärmebrücke (302) dafür gestaltet ist, die durch die Expansion des druckbeaufschlagten Gases erzeugte Kälte zu dem Wärmeleitkissen (304) zu befördern.

6. System gemäß einem der Ansprüche 1 bis 5, wobei die Zusammensetzung eine Pulverphase ist, wie z.B. ein loses Pulver oder ein loses Pulver in einer gasförmigen Phase.

7. System gemäß einem der Ansprüche 1 bis 5, wobei die Zusammensetzung flüssig ist und eine flüssige Phase, ein Gemisch einer flüssigen Phase und einer Pulverphase, ein Gemisch einer flüssigen Phase und einer gasförmigen Phase oder ein Gemisch einer flüssigen, einer Pulver- und einer gasförmigen Phase umfasst, wobei die Zusammensetzung vorzugsweise wenigstens zum Zeitpunkt ihrer Injektion eine Viskosität von höher als oder gleich 1 mPa.s bei 25°C und Atmosphärendruck aufweist.

8. System gemäß Anspruch 7, wobei die Zusammensetzung eine Dispersion von Partikeln in einem flüssigen Medium umfasst, wobei es für die Zusammensetzung möglich ist, über die Aggregation der Partikel fest zu werden, wobei die Zusammensetzung vorzugsweise wenigstens ein Biopolymer, insbesondere ein Alginat, und eine ionische Lösung, insbesondere eine Lösung von zweiwertigen Ionen, umfasst.

9. System gemäß einem der vorstehenden Ansprüche, wobei eine Menge der Zusammensetzung in einer ersten Kammer (5) zwischen einem ersten Kolben (9) und einem Gaspuffer (400) angeordnet ist.

10. System gemäß einem der vorstehenden Ansprüche, wobei die Zusammensetzung wenigstens einen Füllstoff umfasst, insbesondere ein Biopolymer, wie z.B. Hyaluronsäure und/oder eines der Salze davon, Hydroxyapatitpartikel, Polymilchsäure, ein Alginat, Natriummonomethyltrisilanolorthohydroxybenzoat, Collagen, ein Acrylpolymer, wie z.B. Polyacrylamid, ein Methacrylpolymer und/oder Dextran-Mikrokügelchen.

11. System gemäß einem der vorstehenden Ansprüche, umfassend ein Regel- und Steuersystem (140), das dafür gestaltet ist, Gasstöße in die Beförderungskanäle (306; 306a; 306b) abzugeben, wobei das Regel- und Steuersystem (140) Mittel zum Regeln und Steuern des Drucks, der Durchflussrate, der Dauer und/oder Frequenz der abgegebenen Gasstöße umfasst, wobei das Regel- und Steuersystem (140) vorzugsweise ermöglicht, die Injektion der Zusammensetzung in die menschlichen Keratinmaterialien selektiv auszulösen und/oder die menschlichen Keratinmaterialien zu kühlen, und/oder das Regel- und Steuersystem (140) vorzugsweise dafür gestaltet ist, Auslösen einer einzelnen Injektion der Zusammensetzung in die menschlichen Keratinmaterialien oder einer Abfolge oder einer Vielzahl von Abfolgen einer Vielzahl von aufeinanderfolgenden Injektionen zu ermöglichen.

12. Verfahren zur nichttherapeutischen kosmetischen Behandlung von menschlichen Keratinmaterialien, umfassend die folgenden Schritte:
a) Bereitstellen eines nadellosen Injektionssystems (150), umfassend eine zu injizierende Zusammensetzung und ein Reservoir (102), das ein druckbeaufschlagtes Gas enthält, gemäß einem der Ansprüche 1 bis 11,
b) Platzieren des nadellosen Injektionssystems (150) nahe der zu behandelnden menschlichen Keratinmaterialien,
c) Erzeugen von Kälte mithilfe einer Expansion des druckbeaufschlagten Gases,
d) Ausstoßen nach außerhalb des nadellosen Injektionssystems (150), insbesondere Abgeben in die menschlichen Keratinmaterialien, einer Menge der zu injizierenden Zusammensetzung unter Verwendung des Drucks des Gases, wobei das Ausstoßen der Menge der Zusammensetzung nach außerhalb des nadellosen Injektionssystems (150) als ein einzelnes Ausstoßen oder als eine Abfolge oder als eine Vielzahl von Abfolgen einer Vielzahl von aufeinanderfolgenden Injektionen durchgeführt wird,
e) Kühlen der menschlichen Keratinmaterialien mithilfe der durch die Expansion des druckbeaufschlagten Gases erzeugten Kälte.

13. Verfahren gemäß Anspruch 12, wobei Schritt e) vor und/oder während Schritt d) durchgeführt wird oder Schritt e) nach Schritt d) durchgeführt wird.

14. Verfahren gemäß Anspruch 12 oder 13, umfassend die folgenden Schritte:
i. Aufnahme einer Menge der zu injizierenden Zusammensetzung in eine erste Kammer (5) unter einem ersten Kolben (9),
ii. Anheben des ersten Kolbens (9) in die erste Kammer (5), um Umgebungsluft über eine Öffnung (6c) in einem Auslasskanal (6a) einer Injektionsdüse (6) einzuziehen, um einen Luftpuffer (400) in der ersten Kammer (5) unter der in der ersten Kammer (5) vorhandenen Zusammensetzung zu bilden,
iii. Absenken des ersten Kolbens (9) in der ersten Kammer (5), um die in der ersten Kammer (5) vorhandene Zusammensetzung nach außerhalb des nadellosen Injektionssystems (150) durch die Öffnung (6c) in dem Auslasskanal (6a) der Injektionsdüse (6) auszustoßen.

15. Verfahren gemäß einem der Ansprüche 12 bis 14, wobei die menschlichen Keratinmaterialien um wenigstens 3 °C, vorzugsweise wenigstens 5 bis 10°C, relativ zu ihrer Ausgangstemperatur abgekühlt werden.

## Revendications

1. Système d'injection sans aiguille (150) pour injecter une composition, le système (150) comprenant et/ou étant conçu pour recevoir un réservoir (102) contenant un gaz sous pression, notamment stocké sous forme gazeuse et/ou liquéfiée, le système (150) comprenant des conduits d'acheminement (306 ; 306a ; 306b), permettant au gaz d'être dirigé,
d'une part, vers les matières kératiniques humaines afin de refroidir lesdites matières grâce au froid généré par une détente du gaz sous pression et/ou vers une surface (340) configurée de manière à capter le froid généré par une détente du gaz sous pression et à le transmettre vers les matières kératiniques humaines afin de refroidir les matières kératiniques humaines,
et, d'autre part, vers un mécanisme (308 ; 309 ; 10 ; 9) de propulsion de la composition dans les matières kératiniques humaines à l'aide de la pression du gaz.

2. Système selon la revendication 1, comprenant une pièce à main (300) comprenant et/ou étant conçue pour recevoir le réservoir (102) contenant le gaz sous pression.

3. Système selon la revendication 1 ou 2, comprenant un tampon thermique (304) refroidi par le froid généré par la détente du gaz sous pression et configuré de manière à venir s'appuyer sur les matières kératiniques humaines.

4. Système selon la revendication 3, le tampon thermique (304) étant de forme annulaire et l'injection étant réalisée à travers ledit tampon.

5. Système selon la revendication 3 ou 4, comprenant au moins un pont thermique (302) à l'intérieur ou au contact duquel le gaz sous pression se détend, le ou chaque pont thermique (302) étant configuré de manière à acheminer le froid généré par la détente du gaz sous pression vers le tampon thermique (304).

6. Système selon l'une quelconque des revendications 1 à 5, la composition étant une phase pulvérulente, telle qu'une poudre libre, ou une poudre libre dans une phase gazeuse.

7. Système selon l'une quelconque des revendications 1 à 5, la composition étant liquide et comprenant une phase liquide, un mélange d'une phase liquide et d'une phase pulvérulente, un mélange d'une phase liquide et d'une phase gazeuse, ou un mélange d'une phase liquide, d'une phase pulvérulente et d'une phase gazeuse, la composition ayant de préférence, au moins au moment de son injection, une viscosité supérieure ou égale à 1 mPa.s à 25 °C et à la pression atmosphérique.

8. Système selon la revendication 7, la composition comprenant une dispersion de particules en milieu liquide, ladite composition pouvant se solidifier par l'intermédiaire de l'agrégation desdites particules, la composition comprenant de préférence au moins un biopolymère, notamment un alginate, et une solution ionique, notamment une solution d'ions divalents.

9. Système selon l'une quelconque des revendications précédentes, une quantité de la composition étant située dans une première chambre (5) entre un premier piston (9) et un tampon gazeux (400).

10. Système selon l'une quelconque des revendications précédentes, la composition comprenant au moins une charge, notamment un biopolymère tel que l'acide hyaluronique et/ou l'un de ses sels, des particules d'hydroxyapatite, de l'acide polylactique, un alginate, du monométhyltrisilanol orthohydroxybenzoate de sodium, du collagène, un polymère acrylique tel que le polyacrylamide, un polymère méthacrylique et/ou des microsphères de dextrane.

11. Système selon l'une quelconque des revendications précédentes, comprenant un système de régulation et de commande (140) configuré pour délivrer des impulsions de gaz dans les conduits d'acheminement (306 ; 306a ; 306b), le système de régulation et de commande (140) comprenant des moyens de régulation et de commande de la pression, du débit, de la durée et/ou de la fréquence des impulsions de gaz délivrées, le système de régulation et de commande (140) permettant de préférence de déclencher sélectivement l'injection de la composition dans les matières kératiniques humaines et/ou de refroidir les matières kératiniques humaines et/ou le système de régulation et de commande (140) étant de préférence configuré pour permettre le déclenchement d'une seule injection de la composition dans les matières kératiniques humaines ou d'une séquence ou d'une pluralité de séquences d'une pluralité d'injections successives.

12. Procédé de traitement cosmétique non thérapeutique des matières kératiniques humaines, comprenant les étapes suivantes :
a) fournir un système d'injection sans aiguille (150) comprenant une composition à injecter et un réservoir (102) contenant un gaz sous pression, selon l'une quelconque des revendications 1 à 11,
b) placer le système d'injection sans aiguille (150) à proximité des matières kératiniques humaines à traiter,
c) générer du froid au moyen d'une détente du gaz sous pression,
d) éjecter à l'extérieur du système d'injection sans aiguille (150), notamment délivrer dans lesdites matières kératiniques humaines, une quantité de la composition à injecter en utilisant la pression du gaz, l'éjection de ladite quantité de la composition à l'extérieur du système d'injection sans aiguille (150) étant réalisée en une seule éjection ou en une seule séquence ou en une pluralité de séquences d'une pluralité d'injections successives,
e) refroidir lesdites matières kératiniques humaines au moyen du froid généré par la détente du gaz sous pression.

13. Procédé selon la revendication 12, l'étape e) étant réalisée avant et/ou pendant l'étape d), ou l'étape e) étant réalisée après l'étape d).

14. Procédé selon la revendication 12 ou 13, comprenant les étapes suivantes :
i. l'admission d'une quantité de composition à injecter dans une première chambre (5) sous un premier piston (9),
ii. l'élévation du premier piston (9) dans la première chambre (5) de manière à aspirer l'air ambiant par une ouverture (6c) dans un canal de sortie (6a) d'une buse d'injection (6) de manière à former un tampon d'air (400) dans la première chambre (5) sous la composition présente à l'intérieur de la première chambre (5),
iii. l'abaissement du premier piston (9) dans la première chambre (5) de manière à éjecter la composition présente dans la première chambre (5) à l'extérieur du système d'injection sans aiguille (150) par l'ouverture (6c) dans le canal de sortie (6a) de la buse d'injection (6).

15. Procédé selon l'une quelconque des revendications 12 à 14, les matières kératiniques humaines étant refroidies d'au moins 3 °C, de préférence d'au moins 5 à 10 °C, par rapport à leur température initiale.
